**Europäisches Patentamt**

**European Patent Office**

**Office européen des brevets**

(19)

(11) Veröffentlichungsnummer: **0 240 701 B1**

# EUROPÄISCHE PATENTSCHRIFT

(45) Veröffentlichungstag der Patentschrift: **13.11.91**

(51) Int. Cl.5: **A61K 6/04**

(21) Anmeldenummer: **87102843.7**

(22) Anmeldetag: **27.02.87**

(54) **Verwendung einer Legierung zur Herstellung von Gussteilen für die Dentaltechnik.**

(30) Priorität: **19.03.86 DE 3609184**

(43) Veröffentlichungstag der Anmeldung:
**14.10.87 Patentblatt 87/42**

(45) Bekanntmachung des Hinweises auf die
Patenterteilung:
**13.11.91 Patentblatt 91/46**

(84) Benannte Vertragsstaaten:
**AT FR GB SE**

(56) Entgegenhaltungen:
**EP-A- 0 041 938**
**EP-A- 0 164 329**
**DE-C- 3 436 118**
**GB-A- 2 000 188**

(73) Patentinhaber: **Fried. Krupp Gesellschaft mit
beschränkter Haftung
Altendorfer Strasse 103
W-4300 Essen 1(DE)**

(72) Erfinder: **Lindigkeit, Jürgen, Dr.
Mozartstr. 3
W-4690 Herne 2(DE)**

(74) Vertreter: **Vomberg, Friedhelm, Dipl.-Phys.
Schulstrasse 8
W-5650 Solingen 1(DE)**

**Beschreibung**

Es ist bekannt, Gußlegierungen des Typs CoCrMo, wie sie nach ISO 5832/IV international und z.B. nach der ASTM-F-75 in den USA national genormt sind, zur Herstellung chirurgischer Implantate zu verwenden. Die Legierungen dieses Typs haben sich aufgrund ihrer Körperverträglichkeit und ihrer mechanischen Festigkeit als Werkstoffe für Endoprothesen, wie z.B. künstliche Hüft- und Kniegelenke, bewährt.

An Legierungen, die in der Dentaltechnik verwendet werden sollen, werden jedoch im allgemeinen besondere Anforderungen gestellt. Beispielsweise müssen Aufbrennlegierungen für die Metallkeramik mit den handelsüblichen Dentalkeramiken hinsichtlich der thermischen Expansion und Kontraktion kompatibel sein. Außerdem müssen diese Legierungen eine die Haftung zwischen Metall und Keramik gewährleistende Oxidschicht geringer Dikke aufbauen. Auch darf die Oxidfarbe aus ästhetischen Gründen nicht durch die opake Keramik hindurchscheinen. Bei nicht zu verblendenden Dental-Gußstücken, z.B. bei herausnehmbaren Prothesen mit Klammern, wird dazu eine gewisse Aktivierbarkeit und Federhärte verlangt. Besonders wichtig ist in der Dentaltechnik darüber hinaus, daß die Verarbeitung der verwendeten Legierungen mit im Dentallabor zur Verfügung stehenden Mitteln erfolgen kann, sie sollten also mit den üblichen Gußschleudern vergossen werden können. Deshalb enthalten die Legierungen, die bisher in der Dentaltechnik verwendet werden, für die Anwendung als Modellguß-Werkstoff einen weit höheren C-Gehalt als er nach den vorgenannten Normen zulässig ist. Aufbrennlegierungen, die in der Dentaltechnik verwendet werden, basieren meist auf NiCr-Basis, wenn es sich um weitestgehend edelmetallfreie Legierungen handelt.

Es sind auch schon Aufbrennlegierungen auf CoCr-Basis bekannt, vgl. z.B. DE 34 36 118 C1, DE 33 00 909 A1 und EP 0 041 938. Diese Legierungen erfüllen jedoch nicht die Anforderungen, die nach den oben erwähnten Normen an Werkstoffe zur Herstellung chirurgischer Implantate gestellt werden.

So enthält z.B. die Legierung nach EP 0 041 938 0,1 bis 0,25 % C, 0,1 bis 3 % Si und 0,1 bis 8,0 % Mn, wobei die hohen Mn- und Si-Gehalte das Fließverhalten verbessern sollen. Diese hohen Gehalte sind jedoch bei Werkstoffen zur Herstellung von chirurgischen Implantaten, mit denen sich die vorliegende Erfindung befaßt, nicht erwünscht.

Die Erfindung betrifft die Verwendung einer CoCrMo-Guß-Legierung, die 26,5 bis 27,5 % Cr, 4,5 % 5,5 % Mo, 0,65 bis 0,8 % Mn, 0,4 bis 0,5 % Si, höchstens 1 % Fe und 0,05 % C, Rest Co enthält, als Werkstoff zur Herstellung von Gußteilen für Dentalzwecke.

Die Erfindung beruht auf der überraschenden Feststellung, daß Legierungen der angegebenen Zusammensetzung sich auch für die Herstellung von Gußteilen für Dentalzwecke eignen. Überraschend war dabei nicht nur, daß sie trotz ihres geringen Kohlenstoffgehalts ein ausreichendes Fließverhalten aufweisen und ein thermisches Ausdehnungsverhalten haben, das mit Expansionswerten von = $14,5 \times 10^{-6} K^{-1}$ bis $15,5 \times 10^{-6} K^{-1}$ mit den erhältlichen Dentalkeramiken verträglich ist. Auch wurde gefunden, daß diese Legierungen eine ausreichende Oxidschicht bilden, die zu Verbundfestigkeiten beim Schlagversuch von bis zu 42 N führen. Schließlich hat sich gezeigt, daß aus den Legierungen der in den Ansprüchen angegebenen Zusammensetzung sich auch äußerst grazile und dünnwandige Gußteile herstellen lassen, wie sie in der Dentaltechnik häufig vorkommen.

Beispielsweise erreicht man beim Gießen einer Legierung nach Anspruch 2 der Anmeldung in einer induktiv beheizten Laborgußschleuder folgende Werte

| | |
|---|---|
| 0,2 % | Streckgrenze $R_p$ 0,2 = 515 N/mm² |
| | Zugfestigkiet $R_m$ = 740 N/mm² |
| | Bruchdehnung $A_5$ = 18 %. |

Die für Cobalt-Chrom-Legierungen überraschend niedrige Härte der Legierung im gegossenen Zustand beträgt 300 bis 330 HV 10. Sie entspricht damit etwa der Härte des natürlichen Zahnschmelzes, und es kann, da die Härtewerte vergleichbar sind, damit gerechnet werden, daß der natürliche Antagonist nicht abrasiv verschleißt, wie es bei härteren Kronen- und Brückenmaterialien geschieht. Auch ist nicht zu erwarten, daß die Kaufläche von Kronen oder Brücken, die aus Legierungen gemäß der Erfindung hergestellt sind, durch den Antagonisten deformiert wird, wie es bei weicheren Materialien der Fall ist. Ein weiterer Vorteil der erfindungsgemäßen Verwendung der angegebenen Legierung ist der geringe Ni-Gehalt. Er führt dazu, daß auch Ni-allergische Patienten, die Prothesen tragen, die aus den angegebenen Legierungen hergestellt sind, nicht mit Beschwerden rechnen müssen.

**Patentansprüche**

1. Verwendung einer CoCrMo-Guß-Legierung, die 26,5 bis 27,5 % Cr, 4,5 bis 5,5 % Mo, 0,65 bis 0,8 % Mn, 0,4 bis 0,5 % Si, höchstens 1 % Fe und 0,05 % C, Rest Co enthält, als Werkstoff zur Herstellung von Gußteilen für Dentalzwekke.

2. Verwendung einer Legierung nach Anspruch 1, bei der das Verhältnis von Mn zu Si zwischen 1,6:1 und 1,7:1 beträgt, als Werkstoff für die Herstellung von Gußteilen für Dentalzwecke.

3. Verwendung einer Legierung nach Anspruch 1 mit einem Ni-Gehalt zwischen 0,05 und 0,2 % als Werkstoff für die Herstellung von Gußteilen für Dentalzwecke.

## Claims

1. Use of a CoCrMo cast alloy containing 26.5 to 27.5% Cr, 4.5 to 5.5% Mo, 0.65 to 0.8% Mn, 0.4 to 0.5% Si, maximum 1% Fe and 0.05% C, residue Co, as a material for the preparation of castings for dental purposes.

2. Use of an alloy according to claim 1, wherein the Mn/Si ratio is between 1.6 : 1 and 1.7 : 1, as a material for the preparation of castings for dental purposes.

3. Use of an alloy according to claim 1 with a Mi content of between 0.05 and 0.2% as a material for the preparation of castings for dental purposes.

## Revendications

1. Utilisation d'un alliage de coulée CoCrMo, contenant 26,5 à 27,5 % de Cr, 4,5 à 5,5 % de Mo, 0,65 à 0,8 % de Mn, 0,4 à 0,5 % de Si, au plus 1 % de Fe et 0,05 % de C, et, pour le reste, du Co, comme matière pour la fabrication de pièces de fonderie pour usage dentaire.

2. Utilisation d'un alliage suivant la revendication 1, pour lequel le rapport du manganèse au silicium est compris entre 1,6 : 1 et 1,7 : 1, comme matière première pour la fabrication de pièces de fonderie pour usage dentaire.

3. Utilisation d'un alliage suivant la revendication 1, comportant une teneur en nickel comprise entre 0,05 et 0:2 %, comme matière première pour la fabrication de pièces de fonderie pour usage dentaire.